# EUROPEAN PATENT SPECIFICATION

(11) **EP 1 191 939 B1**
(45) Date of publication and mention of the grant of the patent: **17.10.2007**
(21) Application number: 00914980.8
(22) Date of filing: 17.03.2000
(51) Int. Cl.: A61K 38/34, A61K 38/06, A61K 38/08, A61P 13/00, A61P 15/00

(54) **ANTIMICROBIAL AMINO ACID SEQUENCES DERIVED FROM ALPHA-MELANOCYTE-STIMULATING HORMONE**
ANTIMIKROBIELLE AMINOSÄURESEQUENZEN AUS ALPHA-MELANOCYTEN-STIMULIERENDEM HORMON
SEQUENCES D'ACIDES AMINES ANTI-MICROBIENNES TIREES DE L'HORMONE DE STIMULATION DES ALPHA-MELANOCYTES

(30) Priority: 24.03.1999 US 126233 P
(43) Date of publication of application: 03.04.2002
(73) Proprietor: Zengen, Inc., Calabasas, CA 91302 (US)
(72) Inventor: CATANIA, Anna, Pia, I-20122 Milano (IT); LIPTON, James, M., Woodland Hills, CA 91367-4932 (US)
(74) Representative: Viering, Jentschura & Partner
(86) International application number: PCT/US2000/006917
(87) International publication number: WO 2000/059527

(56) References cited:
- US-A- 5 739 111
- CATANIA ANNA ET AL: "Melanocortin peptides inhibit production of proinflammatory cytokines in blood of HIV-infected patients." PEPTIDES (NEW YORK), vol. 19, no. 6, 1998, pages 1099-1104, XP002150797 ISSN: 0196-9781
- CATANIA A ET AL: "The neuropeptide alpha-MSH in HIV infection and other disorders in humans." ANNALS OF THE NEW YORK ACADEMY OF SCIENCES. 1 MAY 1998, vol. 840, 1 May 1998 (1998-05-01), pages 848-856, XP0000944151 ISSN: 0077-8923
- CUTULI ET AL.: 'Antimicrobial effects of alpha-MSH peptides' J. LEUKOCYTE BIOL., vol. 67, no. 2, February 2000, pages 233 - 239, XP002931058
- DEETER ET AL.: 'Antipyretic properties of centrally administered alpha-MSH fragments in the rabbit' PEPTIDES, vol. 9, 1989, pages 1285 - 1288, XP002931079
- HILTZ ET AL.: 'Alpha-MSH peptides inhibit acute inflammation and contact sensitivity' PEPTIDES, vol. 11, no. 5, 1990, pages 979 - 982, XP002931099
- HUANG ET AL.: 'Role of central melanocortins in endotoxin-induced anorexia' AM. J. PHYSIOL., (REGULATORY, INTEGRATIVE AND COMPARATIVE PHYSIOLOGY. 45), vol. 276, no. 3, 1999, pages R864 - R871, XP002931080
- LIPTON ET AL.: 'Mechanisms of antiinflammatory action of the neuro immunomodulatory peptide alpha-MSH' ANNALS OF THE N.Y. ACAD. SCI., vol. 840, 01 May 1998, pages 373 - 380, XP002931100
- RICHARDS ET AL.: 'Effect of alpha-MSH 11-13 (Lysine-proline-valine) on fever in the rabbit' PEPTIDES, vol. 5, 1984, pages 815 - 817, XP002931055
- WEISS ET AL.: 'Corticotropin-peptide regulation of intracellular cyclic-AMP production in cortical neurons in primary culture' J. NEUROCHEM., vol. 45, no. 3, 1985, pages 869 - 874, XP002931056
- GETTING ET AL.: 'POMC gene-derived peptides activate melanocortin type-3 receptor op murine macrophage, suppress cytokine release and inhibit neutrophil migration in acute experimental inflammation' J. IMMUNOL., vol. 162, no. 12, 15 June 1999, pages 7446 - 7453, XP002931098
- HARRIS ET AL.: 'Alpha-melanocyte stimulating hormone (alpha-MSH) and melanin concentrating hormone (MCH) stimulate phagocytosis by head kidney leucocytes of rainbow trout (Oncoryhnchus mykiss) in vitro' FISH & SHELLFISH IMMUNOL., vol. 8, no. 8, November 1998, pages 631 - 638, XP002931054

## Description

### TECHNICAL FIELD

The present invention relates to new pharmaceutical compositions useful as antimicrobial agents, including, for example, for use in reducing the viability of microbes, reducing the germination of yeasts, killing microbes without reducing the killing of microbes by human neutrophils, for treating inflammation in which there is microbial infection without reducing microbial killing, and for increasing the accumulation of cAMP in microbes. More particularly, this invention relates to antimicrobial agents including amino acid sequences derived from alpha-melanocyte-stimulating hormone (α-MSH) and biologically functional equivalents thereof.

### BACKGROUND OF THE INVENTION

Mucosal secretions, phagocytes, and other components of the nonspecific (innate) host defense system initiate the response to microbial penetration before time-consuming adaptive immunity starts. Survival of plants and invertebrates, which lack adaptive immunity, illustrates effectiveness of host defense based on such innate mechanisms.

Endogenous antimicrobial peptides are significant in epithelia, the barrier to environmental challenge that provides the first line of defense against pathogens. Production of natural antimicrobial peptides by phagocytes has been recognized for a long time. These natural antimicrobial peptides generally have a broad spectrum of activity against bacteria, fungi, and viruses. Martin, E., Ganz, T., Lehrer, R.I., Defensins and Other Endogenous Peptide Antibiotics of Vertebrates, J. Leukoc. Biol. 58, 128-136 (1995); Ganz, T., Weiss, J., Antimicrobial Peptides of Phagocytes and Epithelia, Sem. Hematol. 34, 343-354 (1997).

The search for antimicrobial peptides, however, has been painfully difficult and slow. A rare and difficult find has been bactericidal/permeability-increasing protein ("BPI"), which has been used successfully to treat children with severe meningococcal sepsis. Giroir, B.P., Quint, P.A., Barton, P., Kirsh, E.A., Kitchen, L., Goldstein, B., Nelson, B.J., Wedel, N.I., Carrol, S.F., Scannon, P.J., Preliminary Evaluation of Recombinant Amino-terminal Fragment of Human Bactericidal/Permeability-increasing Protein in Children with Severe Meningococcal Sepsis, Lancet 350,1439-1443 (1997).

It would be an important advance in the science to identify the most active amino acid sequences responsible for broad spectrum antimicrobial activity, which would also be useful in new prophylactic and therapeutic antimicrobial treatments.

### SUMMARY OF INVENTION

According to the approach of the invention, the existence of homologs of vertebrate antimicrobial peptides in invertebrates suggests that such peptides are ancestral components of the host defense system. Some of these peptides, or their synthetic homologs, might be suggested for use as therapeutic agents for controlling microbes.

Alpha-melanocyte-stimulating hormone ("α-MSH") is an ancient 13 amino acid peptide produced by post-translational processing of the larger precursor molecule proopiomelanocortin and shares the 1-13 amino acid sequence with adrenocorticotropic hormone ("ACTH"). Eberle, A. N., The Melanotropins, Karger, Basel, Switzerland (1988). α-MSH is known to be secreted by many cell types including pituitary cells, monocytes, melanocytes, and keratinocytes. Lipton, J. M., Catania, A., Anti-inflammatory Influence of the Neuroimmunomodulator α-MSH, Immunol. Today 18, 140-145 (1997). α-MSH occurs in the skin of rats and in the human epidermis. Thody, A.J., Ridley, K., Penny, R.J., Chalmers, R., Fisher, C., Shuster, S., MSH Peptides Are Present in Mammalian Skin, Peptides 4, 813-816 (1983). α-MSH is also found in the mucosal barrier of the gastrointestinal tract in intact and hypophysectomized rats. Fox, J.A.E.T., Kraicer, J., Immunoreactive α-Melanocyte Stimulating Hormone, its Distribution in the Gastrointestinal Tract of Intact and Hypophysectomized Rats, Life. Sci. 28, 2127-2132 (1981). We recently found that human duodenal cells produce α-MSH in culture. Catania et al., unpublished. The presence in barrier organs of this ancient peptide, relatively invariant in amino acid sequence over approximately 300 million years, suggests that it may have a role in the nonspecific (innate) host defense system.

α-Melanocyte-stimulating hormone is known to have potent antipyretic and anti-inflammatory properties. Lipton, J.M., Antipyretic and Anti-inflammatory Lys Pro Val Compositions and Method of Use, U.S. Patent No. 5,028,592, issued July 2, 1991, Lipton, J.M., Antipyretic and Anti-inflammatory Lys Pro Val Compositions and Method of Use, U.S. Patent No. 5,157,023, October 20,1992, Catania, A., Lipton, J. M., α-Melanocyte Stimulating Hormone in the Modulation of Host Reactions, Endocr. Rev. 14, 564-576 (1993); Lipton, J. M., Catania, A., Anti-inflammatory Influence of the Neuroimmunomodulator α-MSH, Immunol. Today 18, 140-145 (1997). α-MSH reduces production of proinflammatory mediators by host cells *in vitro.* Rajora, N., Ceriani, G., Catania, A., Star, R.A., Murphy, M. T., Lipton, J. M., α-MSH Production, Receptors, and Influence on Neopterin, in a Human Monocyte/macrophage Cell Line, J. Leukoc. Biol. 59, 248-253 (1996); Star, R.A, Rajora, N., Huang, J., Stock, R.C., Catania, A., Lipton, J. M., Evidence of Autocrine Modulation of Macrophage Nitric Oxide Synthase by α-MSH, Proc. Natl. Acad. Sci. (USA) 92, 8016-8020 (1995). α-MSH also reduces production of local and systemic reactions in animal models of inflammation. Lipton, J. M., Ceriani, G., Macaluso, A., McCoy, D., Carnes, K., Biltz, J., Catania, A., Anti-inflammatory Effects of the Neuropeptide α-MSH in Acute, Chronic, and Systemic Inflammation, Ann. N. Y. Acad. Sci. 741, 137-148 (1994); Rajora, N., Boccoli, G., Burns, D., Sharma, S., Catania, A., Lipton, J.M., α-MSH Modulates Local and Circulating Tumor Necrosis Factor A in Experimental Brain Inflammation, J. Neurosci. 17, 2181-2186 (1997). The "core" α-MSH sequence (4-10) has learning and memory behavioral effects but little antipyretic and anti-inflammatory activity. Lipton, J. M., Catania, A., Anti-inflammatory Influence of the Neuroimmunomodulator α-MSH, Immunol. Today 18, 140-145 (1997). The active message sequence for these antipyretic and anti-inflammatory activities resides in the C-terminal amino acid sequence of α-MSH, that is, lysine-proline-valine ("Lys-Pro-Val" or "KPV"), which has activities *in vitro* and *in vivo* that parallel those of the parent molecule. Richards, D.B., Lipton, J.M., Effect of α-MSH (11-13) (Lysine-moline-valine) on Fever in the Rabbit, Peptides 5, 815-817 (1984); Hiltz, M. E., Lipton, J.M., Anti-inflammatory Activity of a COOH-terminal Fragment of the Neuropeptide α-MSH, FASEB J. 3, 2282-2284 (1989). These peptides are known to have extremely low toxicity. Lipton, J.M., Catania, A., Anti-inflammatory Influence of the Neuroimmunomodulator α-MSH, Immunol. Today 18, 140-145 (1997).

Melanocortin peptides, including α-MSH, ACTH, and other amino acid sequences derived from α-MSH or ACTH, have heretofore not been studied for potential antimicrobial activity, and there has been no suggestion that melanocortin peptides would have such activity.

According to the invention, it has been determined that α-MSH and certain other amino acid sequences derived from α-MSH have significant antimicrobial uses, including for example, for use in reducing the viability of microbes, reducing the germination of yeasts, killing microbes without reducing the killing of microbes by human neutrophils, for treating inflammation in which there is microbial infection without reducing microbial killing, and increasing the accumulation of cAMP in microbes.

According to a broad aspect of the invention, the antimicrobial agent is selected from the group consisting of one or more peptides including the C-terminal amino acid sequence of α-MSH, that is, KPV, one or more peptides including the amino acid sequence MEHFRWG.

According to one aspect of the invention, the antimicrobial agent is selected from the group consisting of one or more peptides including the C-terminal amino acid sequence of α-MSH, that is, KPV. The KPV sequence is the amino acid sequence α-MSH (11-13). This type of antimicrobial agent includes a dimer of the amino acid sequence KPV, such as VPKCCKPV.

According to a further aspect of the invention, the antimicrobial agent is selected from the group consisting of one or more peptides including the amino acid sequence HFRWGKPV. The HFRWGKPV sequence is the amino acid sequence α-MSH (6-13).

According to a still further aspect of the invention, the antimicrobial agent is selected from the group consisting of one or more peptides including the amino acid sequence SYSMEHFRWGKPV. The SYSMEHFRWGKPV sequence is the entire amino acid sequence of α-MSH (1-13).

According to yet another aspect of the invention, the antimicrobial agent is selected from the group consisting of one or more peptides including the amino acid sequence MEHFRWG. The MEHFRWG sequence is sometimes referred to as the "core" amino acid sequence of α-MSH, that is, α-MSH (4-10).

With these aspects of the invention, it is believed that the shorter amino acid sequences tend to be more effective. Preferably, the antimicrobial agent is further selected from the group consisting of one or more peptides having an amino acid chain length of up to thirteen. Still more preferably, the antimicrobial agent is further selected from the group consisting of one or more peptides having an amino acid chain length of up to eight. Based on the experimental results obtained thus far, the tripeptide KPV is the most effective.

According to the invention, an effective concentration of the antimicrobial agent is at least 10⁻¹² molar, and more preferably the concentration of the antimicrobial agent is at least 10⁻⁶ molar.

It is fully expected that these peptides, which have extremely low toxicity, will be effective in animal and human subjects without adverse effect.

These and other aspects of the invention will be apparent to those persons skilled in the art upon reading the following description of the experimental evidences and discussion.

### BRIEF DESCRIPTION OF THE DRAWING

The accompanying figures of the drawing are incorporated into and form a part of the specification to provide illustrative examples of the present invention and to explain the principles of the invention. The figures of the drawing are only for purposes of illustrating preferred and alternate embodiments of how the invention can be made and used. It is to be understood, of course, that the drawing is intended to represent and illustrate the concepts of the invention. The figures of the drawing are not to be construed as limiting the invention to only the illustrated and described examples. Various advantages and features of the present invention will be apparent from a consideration of the written specification and the accompanying figures of the drawing wherein:
Figure 1 shows the effect of α-MSH (1-13), α-MSH (11-13), and the "KPV dimer" on S. *aureus* colony forming units ("CFU") compared to controls. All three molecules significantly decreased S. aureus colony forming units over a broad range of peptide concentrations.
Figure 2 shows that treatment with urokinase increases S. *aureus* colony formation, but that the addition of α-MSH (1-13) or (11-13) significantly inhibited this urokinase-enhancing effect. *p<0.001 vs urokinase alone.
Figure 3 shows the effect of α-MSH (1-13), α-MSH (11-13), and the "KPV dimer" on *C. albicans* colony forming units ("CFU") compared to controls. All three molecules significantly decreased C. albicans colony forming units over a broad range of peptide concentrations.
Figure 4 shows a comparison of candidacidal activity of certain melanocortin peptides and fluconazole (all 10⁻⁶ M). The most effective of the melanocortin peptides were those including the C-terminal amino acid sequence of α-MSH, for example, α-MSH (1-13), α-MSH (6-13), and α-MSH (11-13).
Figure 5A shows untreated germination of *C. albicans,* i.e, blastospores.
Figure 5B shows horse serum-induced germination of *C. albicans.*
Figure 5C shows the effect of α-MSH (1-13) treatment on germination of *C. albicans.*
Figure 5D shows the effect of α-MSH (11-13) treatment on germination of *C. albicans.*
Figure 6 shows the effect of α-MSH (1-13) and α-MSH (11-13) on *C. albicans* killing by human neutrophils. Values are expressed as percent increase in killing vs medium alone. Scores are means ± SEM.
Figure 7 shows the effect of α-MSH (1-13), α-MSH (11-13), and forskolin on cAMP content of *C*. *albicans.*
Figure 8 shows the inhibitory effect of α-MSH (1-13), α-MSH (11-13), and forskolin on *C*. *albicans* colony forming units.

### DETAILED DESCRIPTION OF THE INVENTION

### I. Materials and Methods

### Peptides

The peptides used in this research included: α-MSH (1-13), (4-10), (6-13), and (11-13), all of which were N-acetylated and C-amidated, and ACTH (1-39) and (18-39) (CLIP). Another peptide used in this research included a dimer of the amino acid sequence KPV, specifically VPKCCKPV, which also was N-acetylated and C-amidated (the "KPV dimer"). The KPV dimer can be chemically represented as NH₂-Lys-Pro-Val-AcCys-CysAc-Val-Pro-Lys-NH₂. The peptides were prepared by solid-phase peptide synthesis and purified by reversed-phase high performance liquid chromatography, as kindly provided by Dr. Renato Longhi, CNR, Milano.

### Organism and culture conditions

*S. aureus* (ATCC 29213) and *C. albicans* (clinical isolate) were obtained from the collection of the Department of Microbiology, Ospedale Maggiore di Milano. *C. albicans* were maintained on Sabouraud's agar slants and periodically transferred to Sabouraud's agar plates and incubated for 48 hours at 28°C. To prepare stationary growth phase yeast, a colony was taken from the agar plate and transferred into 30 ml Sabouraud-dextrose broth and incubated for 72 hours at 32°C. Cells were centrifuged at 1000 x g for 10 minutes and the pellet was washed twice with distilled water. Cells were counted and suspended in Hank's balanced salt solution ("HBSS") to the desired concentration. Viability, determined by the exclusion of 0.01 % methylene blue, remained > 98%.

### Trial of melanocortin peptides on S. aureus viability

*S. aureus* (1x10⁶/ml in HBSS) was incubated in the presence or absence of α-MSH (1-13), α-MSH (11-13), or the "KPV dimer" at concentrations in the range of 10⁻¹⁵ to 10⁻⁴M for 2 hours at 37°C. Cells were then washed in cold distilled water and diluted with HBSS to a concentration of 100 organisms/ml. One ml aliquots were dispensed on blood agar plates and incubated for 24 hours at 37°C. Organism viability was estimated from the number of colonies formed.

In experiments on S. *aureus* we determined the influence of α-MSH on urokinase-induced growth-enhancement. Hart, D.A., Loule, T., Krulikl, W., Reno, C., Staphylococcus Aureus Strains Differ in Their in Vitro Responsiveness to Human Urokinase: Evidence That Methicillin-resistant Strains Are Predominantly Nonresponsive to the Growth-enhancing Effects of Urokinase, Can. J. Microbiol. 42, 1024-31 (1966). *S. aureus* (10⁶ / 100 ml) were incubated for 4 hours at 37 °C with recombinant human urokinase 500 U (Lepetit, Milan, Italy) in a shaking water bath, in the presence or absence of α-MSH (1-13) or (11-13) 10⁻⁶ M. Appropriate dilutions of S. *aureus* were dispensed on agar plates and colonies counted after 24 hours incubation at 37°C.

### Trial of melanocortin peptides on C. albicans viability

*C. albicans* (1 x 10⁶/ml in HBSS) was incubated in the presence or absence of α-MSH (1-13), α-MSH (11-13), or the "KPV dimer" at concentrations in the range of 10⁻¹⁵ to 10⁻⁴M for 2 hours at 37°C. Cells were then washed in cold distilled water and diluted with HBSS to a concentration of 100 organisms/ml. One ml aliquots were dispensed on blood agar plates and incubated for 48 hours at 37°C. Organism viability was estimated from the number of colonies formed.

In subsequent experiments using similar procedures we compared activity of α-MSH (4-10), (6-13), (11-13), ACTH (1-39), (18-39), and fluconazole, the latter being a known antifungal agent. Melanocortin peptides and fluconazole were tested in concentrations of 10⁻⁶ to 10⁻⁴M. There were at least six replicates for each concentration of peptide.

### Trial of α-MSH peptides on C. albicans germination

*C. albicans* from stationary phase cultures were washed twice with distilled water and suspended in HBSS to a final concentration of 2 x 10⁶/ml. Hyphal growth was induced by addition of 10% inactivated horse serum (GIBCO/BRL, Paisley, Great Britain) to yeast incubated for 45 minutes at 37°C with continuous shaking. Horse serum was removed by washing cells twice with HBSS and incubation was continued for 60 minutes at 37°C in the presence of α-MSH (1-13), (6-13), or (11-13) at a concentration of 10⁻⁶ M with continuous shaking. The percentage of filamentous cells was evaluated under a light microscope with the aid of a hemocytometer. Experiments were run in triplicate and at least 200 cells were scored. Photomicrographs were taken with a MC100 camera attached to an Axioskop Zeiss microscope.

### Trial of α-MSH peptides on C. albicans killing by human neutrophils

Venous blood (20 ml) from healthy volunteers was anticoagulated with heparin. Neutrophils were isolated using dextran sedimentation and Ficoll-Hypaque (Sigma Chemical Co., St. Louis, Missouri, USA) centrifugation. Erythrocytes were lysed via hypotonic shock. Neutrophils represented at least 97% of the cell suspension. Cell viability, estimated by trypan blue exclusion, was > 98%. Neutrophils were suspended to final concentration in HBSS.

*C. albicans* (1x10⁶) were opsonized with human AB serum in a shaking water bath for 30 minutes at 37°C. Organisms were then incubated with neutrophils in presence of medium alone or medium with α-MSH (1-13) or α-MSH (11-13) in concentrations of 10⁻¹⁵ to 10⁻⁴ M in a shaking water bath for 2 hours at 37°C. After incubation, the culture tubes were placed on ice to stop growth and extracellular organisms were washed twice with centrifugation at 1000 x g at 4°C. A 2.5% sodium desoxycholate solution was added to the suspension and the tubes were shaken for 5 min. Cold distilled water was added to obtain a suspension of 10⁶ cells/ml. Two 1/100 serial dilution in HBSS were made to obtain a final suspension of 100 cells/ml. Aliquots of 1 ml were dispensed on blood agar plates and incubated for 48 hours at 37°C. Colony forming units ("CFU") were counted at the end of the incubation period. Experiments were run in triplicate and repeated using blood from 5 different donors.

### Trial of α-MSH peptides on cAMP accumulation

*C. albicans* (10⁶/ml), permeabilized with toluene/ethanol, were incubated at 37°C with continuous shaking in the presence of 10⁻⁶ M α-MSH (1-13), (11-13), forskolin, an agent known to increase intracellular cAMP, or in medium alone. The reaction was stopped after 3 minutes by the addition of ice cold ethanol. cAMP was measured in duplicate using a commercial enzyme immunoassay (EIA) kit (Amersham, United Kingdom) after extraction via the liquid-phase method according to manufacturer's instructions. The effect of forskolin (10⁻⁶ M) on *C. albicans* colony formation was determined using the same procedures as for α-MSH peptides.

### Statistical analysis

One-way analysis of variance and Student's *t* test were used to analyze the data. Probability values <0.05 were considered significant.

### II. Results

### α-MSH Peptides inhibited S. aureus colony formation

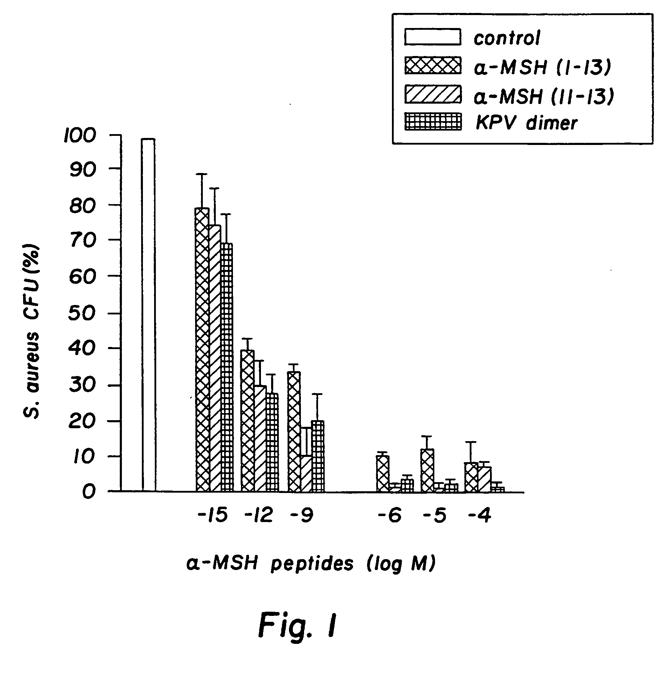

α-MSH peptides (1-13) and (11-13) inhibited *S. aureus* colony formation (Fig. 1). A dimer of the amino acid sequence KPV, specifically, NH₂-Lys-Pro-Val-AcCys-CysAc-Val-Pro-Lys-NH₂, (the "KPV dimer") also inhibited S. aureus colony formation (Fig. 1). The inhibitory effect occurred over a wide range of concentrations and was significant (p<0.01) with peptide concentrations of 10⁻¹² to 10⁻⁴ M.

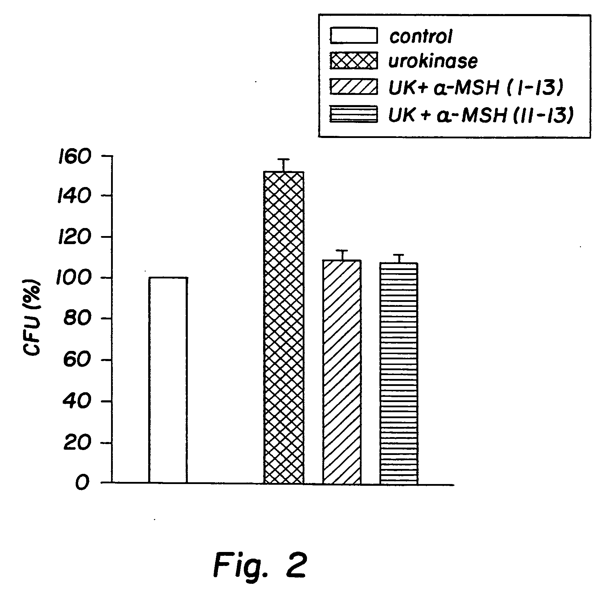

Treatment with urokinase increased *S. aureus* colony formation and addition of α-MSH (1-13) or (11-13) at concentrations of 10⁻⁶ M significantly inhibited the enhancing effect of urokinase (Fig. 2).

### α-MSH Peptides inhibited C. albicans colony formation

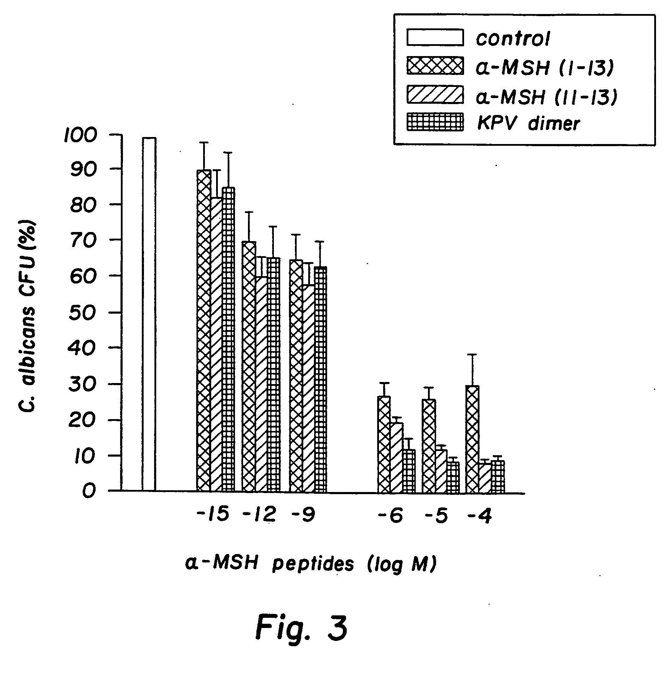

*C. albicans* colony forming units ("CFU') were greatly reduced by α-MSH (1-13) and (11-13) (Fig. 3). A dimer of the amino acid sequence KPV, specifically, KPVCCVPK (the "KPV dimer") also inhibited C. albicans colony formation (Fig. 3). Concentrations of all three peptides from 10⁻¹² to 10⁻⁴ M had significant inhibitory influences on CFU (p<0.01 *vs* control).

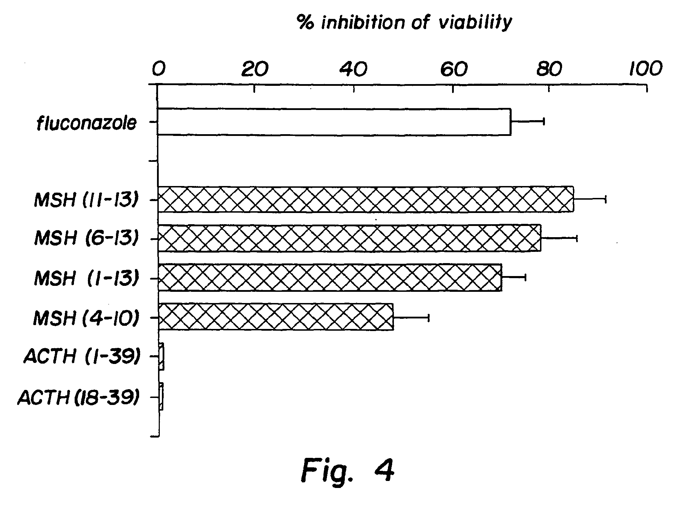

In experiments comparing the relative potency of 10⁻⁶ M melanocortin peptides in reducing *C. albicans* viability, α-MSH (11-13), (6-13), and (1-13) were the most effective (Fig.4). Their inhibitory activity was similar to that of equimolar fluconazole. The "core" α-MSH sequence (4-10), which has behavioral effects but little anti-inflammatory activity, caused approximately 50% inhibition of CFU. Although this inhibitory effect was substantial (p<0.01 *vs* control), it was significantly less than that caused by α-MSH fragments bearing the KPV signal sequence, i.e., α-MSH (6-13) and (11-13) (p<0.01), or the parent molecule α-MSH (1-13) (p<0.05). ACTH (1-39) and the ACTH fragment (18-39) did not reduce *C. albicans* viability (Fig.4). Even higher concentrations of these ACTH peptides (up to 10⁻⁴ M) were likewise ineffective in reducing *C. albicans* CFU (results not shown in the figures).

### α-MSH peptides reduced C. albicans germination

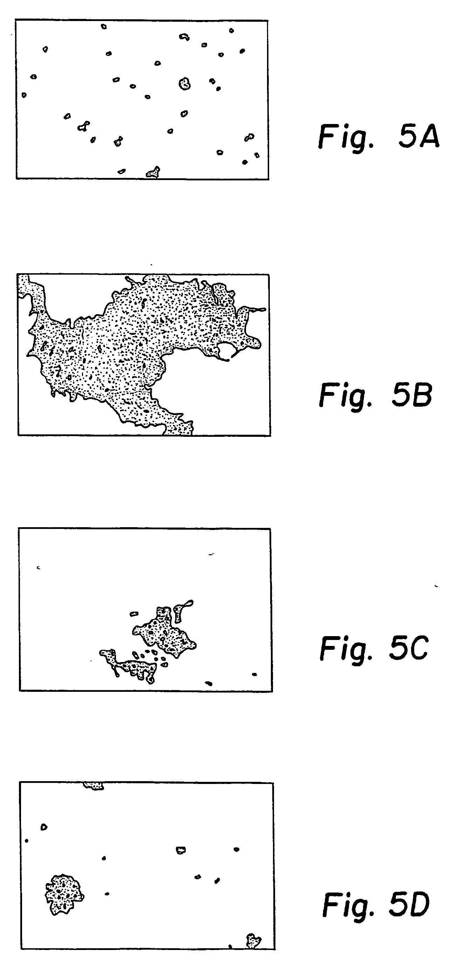

Coincubation of *C. albicans* with α-MSH (1-13) or (11-13) inhibited germ tube formation induced by horse serum (Figs. 5A-D). α-MSH (1-13) caused 28-32% reduction in the number of filamentous cells; the tripeptide inhibited germination by 54-58%. The octapeptide α-MSH (6-13) had similar activity (approximately 50% inhibition) (not shown).

### α-MSH peptides enhanced C. albicans killing by human neutrophils

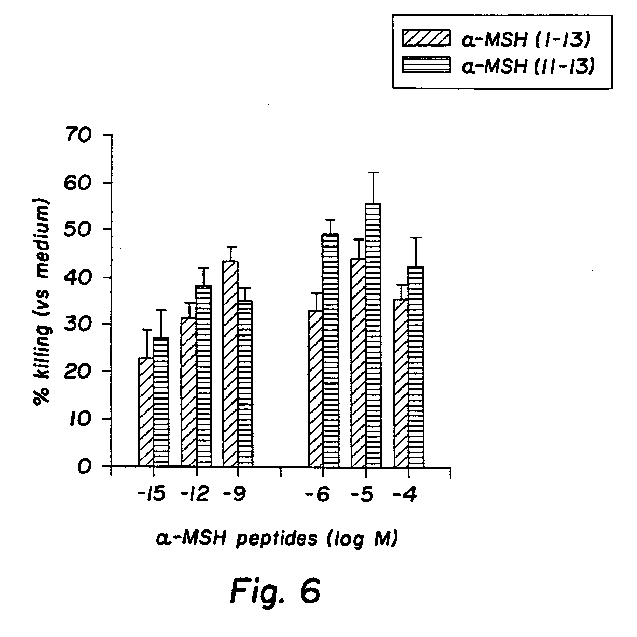

α-MSH (1-13) and (11-13) enhanced killing of *C. albicans* by human neutrophils when administered in concentrations of 10⁻¹² to 10⁻⁴ (p<0.0 1) (Fig.6). Therefore, enhanced killing occurred over a very broad range of concentrations including picomolar concentrations, i.e., the quantity of α-MSH found in human plasma. Catania, A., Airaghi, L., Garofalo, L., Cutuli, M., Lipton, J.M., The Neuropeptide α-MSH in AIDS and Other Conditions in Humans, Ann. N. Y. Acad. Sci. 840, 848-856 (1998).

### α-MSH peptides increased cAMP accumulation

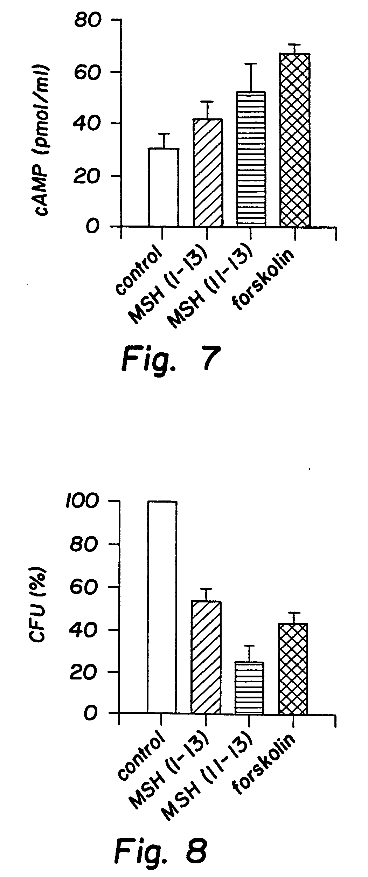

Because many of the effects of α-MSH are known to be mediated by induction of cAMP, we measured effects of α-MSH peptides on cAMP accumulation in *C. albicans.* α-MSH (1-13) and (11-13) enhanced cAMP content in the yeast (Fig.7). The increase was of the same order of magnitude as that induced by equimolar forskolin, an adenylate cyclase activator (Figs. 7). To determine whether increases in cAMP could be responsible for reduction in CFU, we tested the effects of forskolin on *C. albicans* viability. Results showed that 10⁻⁶ M forskolin markedly inhibited *C. albicans* CFU relative to control (p<0.01). The inhibitory effect was similar to that exerted by α-MSH peptides (Fig. 8).

### III. Discussion

### Antimicrobial agents against the viability of microbes

The results show that α-MSH (1-13), its C-terminal tripeptide sequence α-MSH (11-13), and other α-MSH fragments have significant antimicrobial effects against at least two major pathogens: *S*. *aureus* and *C. albicans.* The most effective of the α-MSH peptides were those including the C-terminal amino acid sequence KPV of the α-MSH sequence, i.e., α-MSH (1-13), (6-13), and (11-13). A dimer of the amino acid sequence KPV, specifically, VPKCCKPV (referred to herein as the "KPV dimer") has also been shown to be at least as effective as α-MSH (11-13) against microbes. The α-MSH "core" sequence (4-10), which is known to influence learning and memory, but has little antipyretic and anti-inflammatory influence, was effective, but less so. The ACTH peptides (1-39) and (18-39) did not have significant candidacidal effects. These observations indicate that antimicrobial activity is not common to all melanocortin peptides, but rather that it is specific to α-MSH amino acid sequences, and most particularly to the C-terminal amino-acid sequences of α-MSH.

The antimicrobial effects of these α-MSH peptides occurred over a very broad range of concentrations, including picomolar concentrations that normally occur in human plasma. Catania, A., Airaghi, L., Garofalo, L., Cutuli, M., Lipton, J.M., The Neuropeptide α-MSH in AIDS and Other Conditions in Humans, Ann. N. Y. Acad. Sci. 840, 848-856 (1998). This suggests that endogenous α-MSH has a physiological role in natural immunity.

Therefore, these α-MSH peptides are expected to be useful as a broad prophylactic against microbial infection and in the treatment of human and veterinary disorders resulting from microbial invasion. Further, these peptides that likewise have anti-inflammatory activity could be used to treat cases in which both inflammation and microbial invasion coexist, or where the aim is to prevent their coexistence or development.

### Antimicrobial agents against germination of yeasts

Yeasts can be major pathogens. For example, *C. albicans* is the leading cause of invasive fungal disease in premature infants, diabetics, surgical patients, and patients with human immunodeficiency virus infection or other immunosuppressed conditions. Despite appropriate therapy, death resulting from systemic *C. albicans* infection in immunocompromised patients is substantial. Wenzel, R.P., Pfaller, M.A., Candida Species: Emerging Hospital Bloodstream Pathogens, Infect. Control. Hosp. Epidemiol. 12, 523-4 (1991); Cartledge, J.D., Midgley, J., Gazzard, B.G., Clinically Significant Azole Cross-resistance in Candida Isolates from HIV-Positive Patients with Oral Candidosis, AIDS 11, 1839-44 (1997). The pathogenesis of *C. albicans* infection involves adhesion to host epithelial and endothelial cells and morphologic switching of yeast cells from the ellipsoid blastospore to various filamentous forms: germ tubes, pseudohyphae, and hyphae. Gow, N.A., Germ Tube Growth of Candida Albicans, Curr. Topics Med. Mycol. 8, 43-55 (1997). It is therefore important that α-MSH (1-13) and its C-tetminal tripeptide (11-13) not only reduce the viability of yeast, but also reduce germination of yeast.

### Antimicrobial and anti-inflammation effects without reducing killing by human neutrophils

Reduced killing of pathogens is a dire consequence of therapy with corticosteroids and nonsteroidal anti-inflammatory drugs during infection. Stevens, D.L., Could Nonsteroidal Anti-inflammatory Drugs (NSAIDs) Enhance Progression of Bacterial Infections to Toxic Shock Syndrome?, Clin. Infect. Dis. 21, 977-80 (1997); Capsoni, F., Meroni, P.L., Zocchi, M.R., Plebani, A.M., Vezio, M., Effect of Corticosteroids on Neutrophil Function: Inhibition of Antibody-dependent Cell-mediated Cytotoxicity (ADCC), J. Immunopharmacol. 5, 217-30 (1983). This effect could be particularly dangerous in the immunocompromised host.

α-MSH has potent anti-inflammatory influences in models of acute, chronic, and systemic inflammation. Its wide spectrum of activity and low toxicity suggest that α-MSH is useful for treatment of inflammation in human and veterinary disorders. It was, therefore, important to learn the influence of α-MSH peptides on *C. albicans* killing by phagocytes. This is especially important because α-MSH is known to inhibit neutrophil chemotaxis. Catania, A., Rajora N., Capsoni, F., Minonzio, F., Star, R.A., Lipton, J.M., The Neuropeptide α-MSH Has Specific Receptors on Neutrophils and Reduces Chemotaxis in Vitro, Peptides 17, 675-679 (1996). In the absence of trial, it could have been expected to reduce killing by human neutrophils, despite the direct antimicrobial effect. Results of the present research indicate that α-MSH peptides do not reduce killing but rather enhance it, likely as a consequence of the direct candidacidal effect. Therefore, anti-inflammatory agents such as α-MSH peptides that have antimicrobial effects are expected to be very useful in clinical practice.

### Theoretical discussion and cAMP accumulation

An important question concerns how α-MSH peptides exert their antimicrobial effects and whether they operate like other natural antimicrobial agents.

It is known that α-MSH shares a number of similarities with other natural antimicrobial peptides such as the defensins or the cathelicidins:
1) it is produced in mammals but also in primitive organisms that lack adaptive immunity. Eberle, A. N., The Melanotropins. Karger, Basel, Switzerland (1988).
2) like known antimicrobial peptides, its precursor molecule proopiomelanocortin (POMC) is expressed in phagocytes and epithelia and post-translational proteolytic processing is required to convert it to active α-MSH. Rajora, N., Ceriani, G., Catania, A., Star, R.A., Murphy, M. T., Lipton, J. M., α-MSH Production, Receptors, and Influence on Neopterin, in a Human Monocyte/macrophage Cell Line, J. Leukoc. Biol. 59, 248-253 (1996); Luger, T.A., Schauer, E., Trautinger, F., Krutmann, J., Ansel, J., Schwarz, A., Schwartz, T., Production of Immunosuppressing Melanotropins by Human Keratinocytes, Ann. N. Y. Acad. Sci. 680, 567-570 (1993);
3) it is a cationic peptide; and
4) it has antimicrobial influences against at least two disparate pathogens, a yeast and a bacterium. In addition, α-MSH inhibits HIV-1 replication in acutely and chronically infected monocytes. Barcellini, W., La Maestra, L., Clerici, G., Lipton, J. M., Catania, A., Inhibitory Influences of α-MSH Peptides on Hiv-1 Expression in Monocytic Cells, 12th World AIDS Conference, Geneva, June 28-July 3, 1998. These findings indicate that α-MSH has the broad spectrum of activity of other innate antimicrobial substances.

The mechanism of action of natural antimicrobial agents is only partly understood. Most of these peptides, including the defensins, alter membrane permeability and impair internal homeostasis of the organism. The first contact is made between the cationic groups of the peptide and the negatively charged head of the target membrane. Then, the tertiary structure determines the mode of insertion of the peptide into membranes where they form ion channels or pores that disrupt cell integrity. It is known that cAMP-enhancing agents inhibit mRNA and protein synthesis in *C. albicans.* Bhattacharya, A., Datta, A., Effect of Cyclic AMP on RNA and Protein Synthesis in Candida Albicans, Biochem. Biophys. Res. Commun. 77:1483-44 (1977).

In the present experiments it is shown that α-MSH induces cAMP accumulation in *C. albicans* and also that the cAMP-inducing agent forskolin inhibited colony formation. Without being limited by this theoretical explanation, it may be that the antimicrobial effect was caused by enhancement of this mediator.

### Biologically functional equivalents

As used herein, a biological functional equivalent is defined as an amino acid sequence that is functionally equivalent in terms of biological activity.

Although the specific amino acid sequences described here are effective, it is clear to those familiar with the art that amino acids can be substituted in the amino acid sequence or deleted without altering the effectiveness of the peptides. Further, it is known that stabilization of the α-MSH sequence can greatly increase the activity of the peptide and that substitution of D- amino acid forms for L-forms can improve or decrease the effectiveness of peptides. For example, a stable analog of α-MSH, [Nle⁴,D-Phe⁷]-α-MSH, which is known to have marked biological activity on melanocytes and melanoma cells, is approximately 10 times more potent than the parent peptide in reducing fever. Holdeman, M., and Lipton, J.M., Antipyretic Activity of a Potent α-MSH Analog, Peptides 6, 273-5 (1985). Further, adding amino acids to the C-terminal α-MSH (11-13) sequence can reduce or enhance antipyretic potency (Deeter, L.B., Martin, L.W., Lipton, J.M., Antipyretic Properties of Centrally Administered α-MSH Fragments in the Rabbit, Peptides 9,1285-8 (1989). Addition of glycine to form the 10-13 sequence slightly decreased potency; the 9-13 sequence was almost devoid of activity, whereas the potency of the 8-13 sequence was greater than that of the 11-13 sequence. It is known that Ac-[D-K¹¹]- α-MSH 11-13-NH₂ has the same general potency as the L-form of the tripeptide α-MSH 11-13. Hiltz, M.E., Catania, A., Lipton, J.M., Anti-inflammatory Activity of α-MSH (11-13) Analogs: Influences of Alterations in Stereochemistry, Peptides 12, 767-71, (1991). However, substitution with D-proline in position 12 of the tripeptide rendered it inactive. Substitution with the D-form of valine in position 13 or with the D-form of lysine at position 11 plus the D-form of valine at position 13 resulted in greater anti-inflammatory activity than with the L-form tripeptide. These examples indicate that alterations in the amino acid characteristics of the peptides can influence activity of the peptides or have little effect, depending upon the nature of the manipulation.

It is also believed that biological functional equivalents may be obtained by substitution of amino acids having similar hydropathic values. Thus, for example, isoleucine and leucine, which have a hydropathic index +4.5 and +3.8, respectively, can be substituted for valine, which has a hydropathic index of +4.2, and still obtain a protein having like biological activity. Alternatively, at the other end of the scale, lysine (-3.9) can be substituted for arginine (-4.5), and so on. In general, it is believed that amino acids can be successfully substituted where such amino acid has a hydropathic score of within about +/- 1 hydropathic index unit of the replaced amino acid.

## Claims

1. Use of one or more polypeptides comprising an amino acid sequence selected from the group consisting of KPV, KPVCCVPK, MEHFRWG; HFRWGKPV, SYSMEHFRWGKPV for the manufacture of an antimicrobial pharmaceutical.

2. The use of claim 1, wherein the anti-microbial pharmaceutical does not comprise naturally occurring α-MSH.

3. The use of claim 1 or 2, wherein the antimicrobial pharmaceutical comprises a dimer of two polypeptides comprising the amino acid sequence KPV.

4. The use of any of claims 1 to 3, wherein the one or more peptides have an amino acid chain length of up to thirteen.

5. The use of any of claim 4, wherein the one or more peptides have an amino acid chain length of up to eight.

6. The use of any of claims 1 to 5, wherein the one or more peptides are N-acylated and C-amidated.

7. The use of any of claims 1 to 6, wherein the effective concentration of the one or more peptides is at least 10⁻¹² M.

8. The use of claim 7, wherein the effective concentration of the one or more peptides is at least 10⁻⁶ M.

9. The use of any claims 1 to 5, wherein the one or more peptides has an amino acid sequence selected from the group consisting of KPV, KPVCCVPK, MEHFRWG; HFRWGKPV, SYSMEHFRWGKPV.

10. The use of any of claims 1 to 9, wherein the anti-microbial pharmaceutical has activity against *Staphylococcus aureus* and/or *Candida albicans.*

11. The use of any of claims 1 to 11, wherein the anti-microbial pharmaceutical has antipyretic and/or anti-inflammatory activity.

12. The use of any of claims 1 to 11, wherein the antimicrobial activity is manifested by one or more of the effects selected from the group consisting of:
reducing the viability of microbes;
reducing the germination of yeasts;
killing microbes without reducing the killing of microbes by human neutrophils; treating an inflammation, in which there is a microbial infection without reducing microbial killing; and
increasing the accumulation of cAMP in microbes.

## Patentansprüche

1. Die Verwendung eines oder mehrerer Polypeptide, die eine Aminosäuresequenz umfassen, die aus der Gruppe ausgewählt wird, die aus KPV, KPVCCVPK, MEHFRWG, HFRWGKPV und SYSMEHFRWGKPV besteht, für die Herstellung eines antimikrobiellen Pharmazeutikums.

2. Die Verwendung nach Anspruch 1, wobei das antimikrobielle Pharmazeutikum kein natürlich auftretendes α-MSH umfasst.

3. Die Verwendung nach Anspruch 1 oder 2, wobei das antimikrobielle Pharmazeutikum ein Dimer von zwei Polypeptiden umfasst, das die Aminosäuresequenz KPV umfasst.

4. Die Verwendung nach einem der Ansprüche 1 bis 3, wobei das eine oder die mehreren Peptide eine Aminosäurekettenlänge von bis zu dreizehn aufweisen.

5. Die Verwendung nach Anspruch 4, wobei die einen oder die mehreren Peptide eine Aminosäurekettenlänge von bis zu acht aufweisen.

6. Die Verwendung nach einem der Ansprüche 1 bis 5, wobei das eine oder die mehreren Peptide N-acyliert und C-amidiert sind.

7. Die Verwendung nach einem der Ansprüche 1 bis 6, wobei die wirksame Konzentration des einen oder der mehreren Peptide mindestens 10⁻¹² M ist.

8. Die Verwendung nach Anspruch 7, wobei die wirksame Konzentration des einen oder der mehreren Peptide mindestens 10⁻⁶ M ist.

9. Die Verwendung nach einem der Ansprüche 1 bis 5, wobei das eine oder die mehreren Peptide eine Aminosäuresequenz aufweisen, die aus der Gruppe ausgewählt wird, die aus KPV, KPVCCVPK, MEHFRWG, HFRWGKPV und SYSMEHFRWGKPV besteht.

10. Die Verwendung nach einem der Ansprüche 1 bis 9, wobei das antimikrobielle Pharmazeutikum eine Aktivität gegen *Staphylococcus aureus* und/oder *Candida albicans* aufweist.

11. Die Verwendung nach einem der Ansprüche 1 bis 11, wobei das antimikrobielle Pharmazeutikum eine fiebermildernde und/oder entzündungshemmende Aktivität aufweist.

12. Die Verwendung nach einem der Ansprüche 1 bis 11, wobei sich die antimikrobielle Aktivität durch eine oder mehrere der Wirkungen manifestiert, die aus der Gruppe ausgewählt werden, die besteht aus:
Verringern der Lebensfähigkeit von Mikroben;
Verringern der Keimung von Hefen;
Töten von Mikroben ohne Verringern des Tötens von Mikroben durch humane Neutrophile; Behandeln einer Entzündung, in der es eine mikrobielle Infektion gibt ohne Verringern der mikrobiellen Tötung; und
Erhöhen der Akkumulation von cAMP in Mikroben.

## Revendications

1. Utilisation d'un ou plusieurs polypeptides comprenant une séquence d'acides aminés choisie dans le groupe constitué de KPV, KPVCCVPK, MEHFRWG ; HFRWGKPV, SYSMEHFRWGKPV pour la fabrication d'un produit pharmaceutique antimicrobien.

2. Utilisation selon la revendication 1, dans lequel le produit pharmaceutique antimicrobien ne comprend pas d'α-MSH naturelle.

3. Utilisation selon la revendication 1 ou 2, dans laquelle le produit pharmaceutique antimicrobien comprend un dimère de deux polypeptides comprenant la séquence d'acides aminés KPV.

4. Utilisation selon l'une quelconque des revendications 1 à 3, dans laquelle les un ou plusieurs peptides ont une longueur de chaîne d'acides aminés allant jusqu'à treize acides aminés.

5. Utilisation selon la revendication 4, dans laquelle les un ou plusieurs peptides ont une longueur de chaîne d'acides aminés allant jusqu'à huit acides aminés.

6. Utilisation selon l'une quelconque des revendications 1 à 5, dans laquelle les un ou plusieurs peptides sont N-acylés et C-amidés.

7. Utilisation selon l'une quelconque des revendications 1 à 6, dans laquelle la concentration efficace des un ou plusieurs peptides est d'au moins 10⁻¹² M.

8. Utilisation selon la revendication 7, dans laquelle la concentration efficace des un ou plusieurs peptides est d'au moins 10⁻⁶ M.

9. Utilisation selon l'une quelconque des revendications 1 à 5, dans laquelle les un ou plusieurs peptides ont une séquence d'acides aminés choisie dans le groupe constitué de KPV, KPVCCVPK, MEHFRWG ; HFRWGKPV, SYSMEHFRWGKPV.

10. Utilisation selon l'une quelconque des revendications 1 à 9, dans laquelle le produit pharmaceutique antimicrobien a une activité contre *Staphylococcus aureus* et/ou *Candida albicans.*

11. Utilisation selon l'une quelconque des revendications 1 à 11, dans laquelle le produit pharmaceutique antimicrobien a une activité antipyrétique et/ou anti-inflammatoire.

12. Utilisation selon l'une quelconque des revendications 1 à 11, dans laquelle l'activité antimicrobienne se manifeste par un ou plusieurs des effets choisis dans le groupe constitué de :
réduction de la viabilité des microbes;
réduction de la germination des levures;
destruction des microbes sans réduction de la destruction des microbes par les neutrophiles humains ; traitement d'une inflammation, dans laquelle il y a une infection microbienne sans réduction de la destruction des microbes ; et
augmentation de l'accumulation de AMPc dans les microbes.
